# EUROPEAN PATENT SPECIFICATION

(11) **EP 0 612 255 B1**
(45) Date of publication and mention of the grant of the patent: **05.06.1996**
(21) Application number: 92924149.5
(22) Date of filing: 04.11.1992
(51) Int. Cl.: A61M 5/28, A61M 5/34

(54) **AN INJECTION DEVICE**
INJEKTIONSVORRICHTUNG
DISPOSITIF D'INJECTION

(30) Priority: 15.11.1991 AU 9506/91
(43) Date of publication of application: 31.08.1994
(73) Proprietor: DELTA WEST PTY. LIMITED, Bentley, W.A. 6102 (US)
(72) Inventor: UNSWORTH, Rodney, William, Dalkeith, W.A. 6009 (AU); CARTER, Stephen, John, Kingsley, W.A. 6026 (AU)
(74) Representative: Perry, Robert Edward
(86) International application number: US9209260
(87) International publication number: WO9309826

(56) References cited:
- EP-A- 0 356 679
- FR-A- 2 089 176
- US-A- 2 828 743
- US-A- 4 568 336

## Description

The present invention relates to an injection device for medical or veterinary injection. It will be convenient to describe the invention in relation to injection devices for self injection by relatively unskilled operators to treat medical conditions although it will be appreciated that the invention has much wider application.

Numerous medical conditions require treatment of a patient by self injection of drug, for example insulin-dependant diabetes is generally treated by self injection of insulin; male erectile impotence is often treated by way of intracavernosal injection of erectile impotence treating drugs. In both the above medical conditions self injection is generally by way of insulin syringes. These syringes are long and thin and usually have a fine gauge needle already attached to the syringe during manufacture. While such needles may be adequate for young dextrous hands which can manipulate this type of syringe, they can be cumbersome and very difficult to handle by older or infirm persons with limited coordination and who may also be afflicted with complaints such as arthritis which may further effect dexterity. Moreover, with the insulin type syringe, it is necessary for the user to have a relatively wide extension between the thumb and index finger to manipulate and actuate the syringe properly. This may cause a problem with older users who cannot easily extend their thumb or index finger across a broad extension to manipulate an insulin type syringe.

Other risks are associaterd with injection of substances. First, particularly in cases of intravenous injection there exists the danger of injecting air and causing embolism. Secondly, there is a danger of drawing up physiological fluids or the drug just injected upon completion of injection or if the injecting pressure is released during injection.

Furthermore there is nearly always a risk of contamination of the injection apparatus by handling of exposure to the environment. This risk is not only present where an unskilled operator is involved, but also where a skilled practitioner is acting in adverse conditions such as accident or emergency scenes or war zones.

US-A-2828743 discloses a snap-on cartridge needle unit in which a hub or stopper engages with a rigid syringe body, for use with a non-deformable ampoule. EP-A-0356679 describes a corresponding device, incorporating a deformable ampoule. In each case, the needle-connecting assembly is secured to the ampoule on the outside of the ampoule's neck.

According to the present invention, an injection device comprises a deformable ampoule having a neck portion to which is attached a body having a continuous fluid conduit extending therethrough and a distal injection needle, and a one-way valve in the conduit, and is characterised in that the body also includes an integral proximal hollow male frustoconical member having an outer surface in sealing engagement with an inner surface of the ampoule's neck portion, that the body further comprises a proximal annular collar separate from and surrounding the male frustoconical member and having an inner surface in engagement with an outer surface of the ampoule's neck portion, and that the body also comprises a distal annular collar separate from and surrounding the needle, for limiting the depth of insertion of the needle.

The novel device is suitable for use in self-injection by inexperienced patients or by patients with limited flexibility and coordination. Further, the novel device can reduce the possibility of contamination during handling, reduce or eliminate the risk of injecting air and causing an embolism in the patient, and reduce the risk of drawing up physiological fluids during or upon completion of injection. Such a device should be simple to use and inexpensive to produce.

The injection device is adapted to engage with and form a fluid-tight seal with a deformable plastic ampoule so that, after the injection device and ampoule are cooperatively engaged, the needle is inserted into the patient and the ampoule is squeezed to express its contents through the needle into the patient.

The body of the injection device may be any suitable size or shape but is preferably of a suitable size to allow easy manipulation, especially by an inexperienced or elderly patient. At one end of the body there is located an injection needle having a fluid conduit passing therethrough, the needle of the kind well known in the art. Different devices made in accordance with the present invention may utilize different types of needles, depending upon the intended use of the device. For example if the device is for intercavernosal injection, a very fine grade needle is utilized, whereas if the device is for deep intramuscular injection, a thick, longer needle will be utilized. Preferably the needle is permanently connected to the body when the body is manufactured. A removable protective cap is preferably provided to cover the needle until the device is required for use. The protective cap may be a screw-on cap which engages with threads adjacent the needle, or the cap may engage an area adjacent the needle by friction fit or the like.

The male frusto-concial extending member at the other end of the body is adapted to engage a corresponding parallel walled or frusto-conical female recess on an ampoule. Preferably the fitting between the male member and female recess is a luer, which comprises a male cone with a conicity of 6:100 cooperating with a corresponding female cone on the ampoule. However, other conicities may be suitable provided that a fluid-tight seal is formed when the ampoule is engaged with the injection device.

When the injection device and deformable ampoule are connected, importantly the male member is inserted into the neck of the ampoule which has either a parallel walled or frusto-conical shaped recess in the neck. The ampoule is generally of the kind being blow-moulded from a thermoplastics material such as polypropylene, polyethylene or a blend thereof as is commonly used in the art, and having a cap portion joined to the neck of the ampoule by a frangible web or the like. An advantage of the present invention is that once the ampoule is opened by snapping off the cap, the surface of the ampoule which mates with the injection device is on the inside of the ampoule, rather than on the outside of the ampoule which is the case with known injection devices. The engagement with the inside of the ampoule significantly reduces the risk of contamination as a result of handling of the ampoule, as the surfaces of the ampoule exposed to the fingers of the user do not come into contact with the fluid conduit through the injection device, and do not form part of the sealing surfaces with the injection device.

The injection device is provided with an annular collar radially spaced from and at least partially surrounding the male extending member. The annular collar may operate as a shield to protect the male member from contact with the user's fingers and hence protects from the risk of contamination. The annular collar must be suitably spaced from the male member to allow sufficient clearance for the neck portion of the ampoule when positioned over the male member. Preferably the annular collar does not totally surround the male member such that a part of the male member protrudes from the collar to facilitate location of the ampoule about the male member.

In a preferred embodiment the annular collar includes one or more barbs which project inwardly towards the male member. Upon engagement of the ampoule with the male member the barb or barbs engage the outside of the neck portion of the ampoule to prevent removal of the ampoule. In one embodiment, when the male member is of the luer lock type and it is necessary to twist the ampoule onto the injection device, the barb(s) are configured to prevent unscrewing of the ampoule from the device. The barbs will preferably deform the neck of the ampoule by cutting or bitting into the neck or otherwise scoring the neck when the ampoule is twisted onto the injection device. Similarly, if a luer slip type connection is present between the ampoule and injection device, such deformation of the ampoule neck may occur when the ampoule is simply pushed onto the male member. The advantage of such non-removable engagement between the injection device and ampoule is that the device is capable of being used only once, and therefore the risk of cross-infection from multiple uses is eliminated.

The barb(s) preferably have a surface distal the body of the injection device which slope inward toward the body. These sloping surfaces assist in locating the neck of the ampoule about the device and act as guides to centre the ampoule neck about the male member. The sloping surfaces also assist in enabling the ampoule to be pushed onto the injection device.

Preferably two barbs are provided on the collar the barbs being positioned opposite each other on the collar. Cut-out portions may be provided in the collar, preferably adjacent each barb.

The configuration of the present invention further improves over the converse arrangement known in the prior art i.e. where an ampoule has a male neck insertable into a female recess in the injection device. In the present invention where the ampoule is made from a relatively pliable thermoplastic material and the majority of the injection device is made from a rigid thermoplastic material the locking mechanism between the injection device and ampoule is considerably more effective as the locking barbs are formed on the injection device from the relatively rigid material, rather than on the ampoule from a softer material; the latter arrangement not being as secure as the former.

In another embodiment of the present invention there may be provided a valve means in the fluid conduit between the male member and needle. The valve may be any suitable valve means known in the art and may be positioned to permit flow of fluid along the fluid conduit from the male member to the needle, but to prevent flow from the needle back to the male member. Particularly in relation to intracavernosal self-injection it is undesirable for physiological fluids to be drawn back into the injection device and the valve means is adapted to prevent such back flow. In some applications however, such as dental injection, it is desirable to withdraw physiological fluids into the injection device to ascertain whether or not a blood vessel has been struck. In such applications the valve means will not be present, and preferably the injection device will be made from a transparent material so that the colour of any physiological fluid withdrawn can be noted.

In another aspect of the present invention there is provided stop means to limit penetration of the needle into the patient. Particularly in cases of self injection it is desirable that the needle penetrate the patient only to a limited extent. Preferably the stop means comprises a flange which extends from the body to allow a predetermined length of the needle to protrude from the body beyond the flange. During injection the needle is inserted into a patient, to a point where the flange abuts the skin of the patient, preventing further insertion.

The injection device of the present invention is suitable for but not limited to intramuscular, intravenous, subcutaneous and intracavernosal injection although it may also be suitable for blood-taking if the ampoule is suitably configured to allow a vacuum to be applied to the neelde. The ampoule adapted for use with the present invention may contain any suitable substance for injection, including drug solutions such as narcotics, anaesthetics and hormones etc., sodium chloride solutions, potassium chloride solutions, water or the like.

### DESCRIPTION OF THE DRAWINGS

It is to be understood that the drawings and following description relate to a preferred embodiment only, and are not intended to limit the scope of the invention.

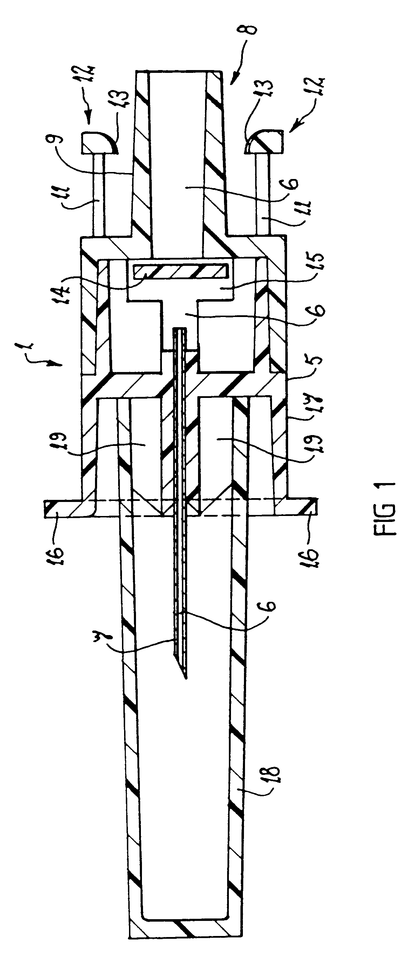

Figure 1 is an axial cross-section of an injection device made in accordance with the present invention.

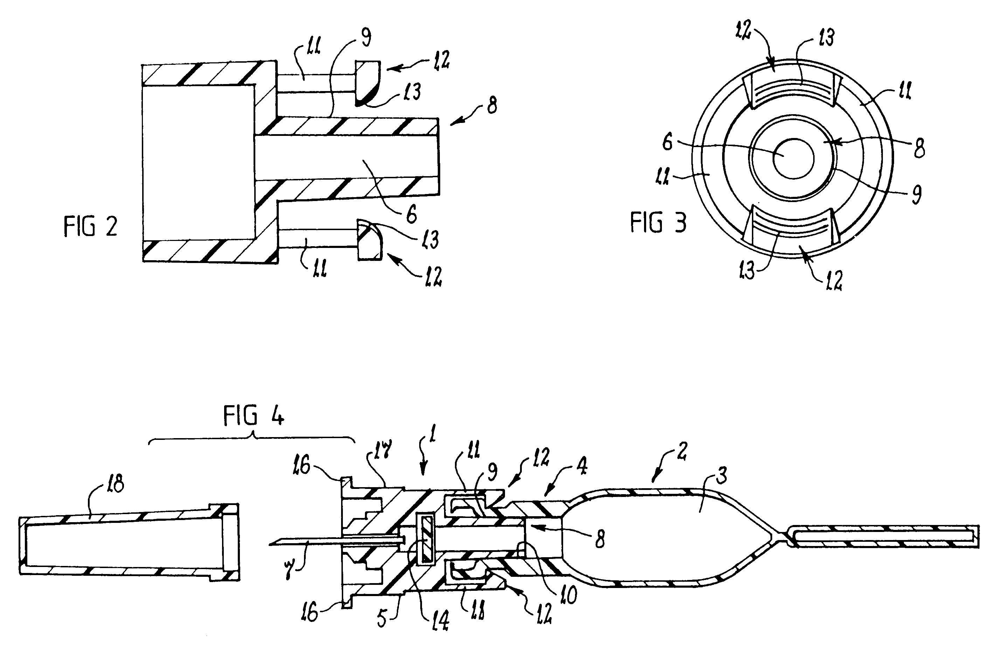

Figure 2 is an axial cross-section of the ampoule engaging end of an injection device of Figure 1.

Figure 3 is an end elevation of an injection device of Figure 1 showing the ampoule engaging end.

Figure 4 is an axial cross-section of an injection device of Figure 1 with an ampoule attached.

Injection device 1 is adapted to engage a deformable plastic ampoule 2 having a reservoir 3 and a neck portion 4. The injection device 1 has a body 5 having a continuous fluid conduit 6 therethrough. The continuous fluid conduit comprises at one end an injection needle 7 and at the other end a male frusto-conical extending member 8 which tapers away from body 5. The outer surface of member 8 forms a sealing surface with a corresponding frusto-conical female recess 10 on the neck portion 4 of ampoule 2.

Male member 8 and conical female recess 10 are preferably cooperating male and female luer cones.

An annular collar 11 spaced from member 8 is provided which at least partially surrounds member 8 and reduces the risk of member 8 from being touched by the fingers of the user. Collar 11 includes a plurality of barbs 12 which project inwardly towards member 8 and which engage the outside of the neck portion 4 of the ampoule 2. The surface 13 of each barb 12 which is distal the body 5 slopes inwardly towards the body to facilitate easy coupling of the ampoule 2 and injection device 1.

The portions of collar adjacent each barb 8 may be cut-out (not shown).

Valve member 14 may be provided in fluid conduit 6 in a valve chamber 15. Valve member 14 may be a resilient slideable plug or the like.

Stop means 16 comprises an annular flange extending from body 5 on an annular collar 17. Stop means 16 allows a predetermined length of needle 7 to extend beyond the flange.

A protective cap 18 may be provided to protect needle 7. Cap 18 may engage members 19 by friction fit and can be removed and replaced easily by the user.

In use ampoule 2 is opened by removing the frangible cap covering neck 4, thus exposing female recess 10. Neck portion 4 is brought into contact with and centered about male member 8 and fitted between male member 8 and annular collar 11. The end of neck 4 is guided into a central position by the sloping surfaces 13 of each barb 12.

The ampoule 2 and injection device 1 are then pushed together such that surfaces 9 and 10 engage and seal against one another. If the injection device 1 comprises a luer slip fitting, ampoule 2 is simply pushed onto the male member 8, but if the injection device has a liner lock fitting, ampoule 2 is pushed and twisted onto male member 8.

In the process of connecting the ampoule 2 and injection device 1 barbs 12 grip and deform neck 4 so that the ampuole cannot readily be removed once attached. Any resilience in neck 4 will allow a tight seal between female recess 10 and male member 8.

Cap 18 is then removed and needle 7 is inserted into the patient until the skin of the patient comes into contact with stop means 16 such that further penetration of the needle is prevented. The user then squeezes the side walls of ampoule 2 to eject fluid from the reservoir 3 through fluid conduit 6, past valve 14 (where present) and out of needle 7.

Where valve 14 is present, backflow of physiological fluid towards ampoule 2 is prevented when valve 14 seals the fluid conduit 6.

Upon complete discharge of the ampoule's contents, the needle 7 is withdrawn and cap 18 is replaced. The spent injection device and ampoule is then disposed of in an appropriate manner.

The injection device may be made from any suitable material, preferably a sterilizable thermoformable plastic material such a polycarbonate. The invention is particularly suitable when the ampoule is made from a pliable polypropylene, polyethylene or a blend thereof as is commonly used in the art. The injection device may be manufactured by any suitable means known to those skilled in the art such as injection moulding or the like.

It is to be understood that various modifications, additions and/or alterations may be made to the parts previously described without departing from the scope of the present invention, as defined by the following claims.

## Claims

1. An injection device comprising a deformable ampoule (2) having a neck portion (4) to which is attached a body (5) having a continuous fluid conduit (6) extending therethrough and a distal injection needle (7), and a one-way valve in the conduit (6), characterised in that the body (5) also includes an integral proximal hollow male frustoconical member (8) having an outer surface (9) in sealing engagement with an inner surface (10) of the amouple's neck portion (4), that the body (5) further comprises a proximal annular collar (11) separate from and surrounding the male frustoconical member (8) and having an inner surface (13) in engagement with an outer surface of the ampoule's neck portion (4), and that the body (5) also comprises a distal annular collar (17) separate from and surrounding the needle (7), for limiting the depth of insertion of the needle.

2. The injection device of claim 1, wherein the proximal annular collar (11) comprises one or more barbed members (12) for engagement with the outer surface of the ampoule's neck portion (4).

3. The injection device of claim 2, wherein the or each barbed member (12) has an inner surface (13) which slopes radially inwards towards the body (5).

4. The injection device of claim 2, wherein the proximal annular collar (11) includes one or more cut-out portions adjacent to the or each barbed member (12).

5. The injection device of any of claims 2 to 4, which comprises two barbed members (12), mounted opposite to each other on the proximal annular collar (11).

6. The injection device of claim 1, wherein the outer surface of the ampoule neck portion (4) and the inner surface (13) of the proximal annular collar (11) comprise cooperative liner fittings.

## Patentansprüche

1. Injektionsvorrichtung, umfassend eine deformierbare Ampulle (2) mit einem Halsteil (4), an dem ein Körper (5) mit einer ihn durchsetzenden, durchgehenden Fluidleitung (6) und einer distalen Injektionsnadel (7) angebracht ist, und ein in der Leitung (6) angeordnetes Einwegeventil, dadurch gekennzeichnet, daß der Körper (5) ferner ein materialeinheitlich geformtes, proximales, hohles kegelstumpfförmiges Steckglied (8) mit einer Außenfläche (9), die in Dichtungsberührung mit einer Innenfläche (10) des Ampullen-Halsteils (4) steht, aufweist, daß der Körper (5) weiterhin einen vom kegelstumpfförmigen Steckglied (8) getrennten (beabstandeten) und dieses umgebenden ringförmigen Kragen (11) mit einer Innenfläche (13), die an einer Außenfläche des Ampullen-Halsteils (4) anliegt, aufweist und daß der Körper (5) zudem mit einem von der Nadel (7) getrennten (beabstandeten) und diese umgebenden, distalen ringförmigen Kragen (17) zum Begrenzen der Einführtiefe der Nadel versehen ist.

2. Injektionsvorrichtung nach Anspruch 1, wobei der proximale ringförmige Kragen (11) ein oder mehrere (wider)-hakenartige Glieder (12) für einen Eingriff mit der Außenfläche des Ampullen-Halsteils (4) aufweist.

3. Injektionsvorrichtung nach Anspruch 2, wobei das oder jedes hakenartige Glied (12) eine radial einwärts in Richtung auf den Körper (5) geneigte oder abgeschrägte Innenfläche (13) aufweist.

4. Injektionsvorrichtung nach Anspruch 2, wobei der proximale ringförmige Kragen (11) neben dem oder jedem hakenartigen Glied (12) einen oder mehrere Ausschnittbereiche aufweist.

5. Injektionsvorrichtung nach einem der Ansprüche 2 bis 4 umfassend zwei einander gegenüberliegend am proximalen ringförmigen Kragen (11) angebrachte hakenartige Glieder (12).

6. Injektionsvorrichtung nach Anspruch 1, wobei die Außenfläche des Ampullen-Halsteils (4) und die Innenfläche (13) des proximalen ringförmigen Kragens (11) miteinander zusammenwirkende Luersche Kupplungen (fittings) bilden.

## Revendications

1. Dispositif d'injection comprenant une ampoule déformable (2) ayant une partie de col (4) à laquelle est fixé un corps (5) ayant un conduit continu pour fluide (6) passant par lui et une aiguille distale d'injection (7), ainsi qu'un clapet unidirectionnel disposé dans le conduit (6), caractérisé en ce que le corps (5) comprend aussi un élément tronconique mâle proximal creux (8) qui est monobloc avec lui et qui a une surface extérieure (9) en contact d'étanchéité avec une surface intérieure (10) de la partie de col (4) de l'ampoule, en ce que le corps (5) comprend par ailleurs un collet annulaire proximal (11) séparé de et entourant l'élément tronconique mâle (8) et ayant une surface intérieure (13) en appui contre une surface extérieure de la partie de col (4) de l'ampoule et en ce que le corps (5) comprend également un collet annulaire distal (17) séparé de et entourant l'aiguille (7) pour limiter la profondeur de pénétration de l'aiguille.

2. Dispositif d'injection selon la revendication 1, dans lequel le collet annulaire proximal (11) comprend un ou plusieurs éléments barbelés (12) destinés à s'appliquer contre la surface extérieure de la partie de col (4) de l'ampoule.

3. Dispositif d'injection selon la revendication 2, dans lequel le ou chaque élément barbelé (12) comporte une surface intérieure (13) qui est en pente radialement vers l'intérieur et vers le corps (5).

4. Dispositif d'injection selon la revendication 2, dans lequel le collet annulaire proximal (11) comprend une ou plusieurs parties découpées qui sont voisines de ou de chaque élément barbelé (12).

5. Dispositif d'injection selon l'une quelconque des revendications 2 à 4, qui comprend deux éléments barbelés (12) montés en face l'un de l'autre sur le collet annulaire proximal (11).

6. Dispositif d'injection selon la revendication 1, dans lequel la surface extérieure de la partie de col (4) de l'ampoule et la surface intérieure (13) du collet annulaire proximal (11) comprennent des joints coopérant à emboîtement.
